# EUROPEAN PATENT SPECIFICATION

(11) **EP 2 916 130 B1**
(45) Date of publication and mention of the grant of the patent: **25.05.2022**
(21) Application number: 13851081.3
(22) Date of filing: 01.11.2013
(51) Int. Cl.: G01N 30/02, G01N 30/72, G01N 33/15

(54) **METHOD FOR ESTABLISHING SHENQI FUZHENG INJECTION FINGERPRINT SPECTRUM**
VERFAHREN ZUR HERSTELLUNG EINES SHENQI-FUZHENG-INJEKTIONS-FINGERABDRUCKSPEKTRUMS
PROCÉDÉ D'ÉTABLISSEMENT DE SPECTRE D'EMPREINTES DIGITALES D'INJECTION DE SHENQI FUZHENG

(30) Priority: 02.11.2012 CN 201210436493
(43) Date of publication of application: 09.09.2015
(73) Proprietor: Li Min Pharmaceutical Factory of Livzon Pharmceutical Group, Shaoguan Guangdong 512028 (CN)
(72) Inventor: SONG, Yangang, Shaoguan Guangdong 512028 (CN); LIU, Xuehua, Shaoguan Guangdong 512028 (CN); HUANG, Wenhua, Shaoguan Guangdong 512028 (CN); LIU, Donglai, Shaoguan Guangdong 512028 (CN)
(74) Representative: Schiweck Weinzierl Koch Patentanwälte Partnerschaft mbB
(86) International application number: PCT/CN2013/086405
(87) International publication number: WO 2014/067478

(56) References cited:
- CN-A- 1 899 362
- CN-A- 101 088 520
- CN-A- 101 352 478
- CN-A- 102 028 840
- CN-A- 102 621 244
- GB-A- 2 455 151
- US-A1- 2006 292 246
- JIANGHAO SUN ET AL: "Differentiation ofgrown in the USA and China using LC/MS-based chromatographic fingerprinting and chemometric approaches", ANALYTICAL AND BIOANALYTICAL CHEMISTRY, SPRINGER, BERLIN, DE, vol. 399, no. 5, 13 January 2011 (2011-01-13), pages 1877-1889, XP019876190, ISSN: 1618-2650, DOI: 10.1007/S00216-010-4586-7
- CHRISTOPHE TISTAERT ET AL: "Chromatographic separation techniques and data handling methods for herbal fingerprints: A review", ANALYTICA CHIMICA ACTA, ELSEVIER, AMSTERDAM, NL, vol. 690, no. 2, 9 February 2011 (2011-02-09), pages 148-161, XP028369523, ISSN: 0003-2670, DOI: 10.1016/J.ACA.2011.02.023 [retrieved on 2011-02-16]
- ZHAO L ET AL: "Fingerprint analysis of Psoralea corylifolia L. by HPLC and LC-MS", JOURNAL OF CHROMATOGRAPHY B: BIOMEDICAL SCIENCES & APPLICATIONS, ELSEVIER, AMSTERDAM, NL, vol. 821, no. 1, 5 July 2005 (2005-07-05), pages 67-74, XP027627270, ISSN: 1570-0232 [retrieved on 2005-07-05]
- DONG JU ET AL: "Shenqi fuzheng, an injection concocted from chinese medicinal herbs, combined with platinum-based chemotherapy for advanced non-small cell lung cancer: a systematic review", JOURNAL OF EXPERIMENTAL & CLINICAL CANCER RESEARCH, BIOMED CENTRAL LTD, LONDON UK, vol. 29, no. 1, 22 October 2010 (2010-10-22), page 137, XP021083421, ISSN: 1756-9966, DOI: 10.1186/1756-9966-29-137
- XIA, YUN ET AL.: 'Analysis of Chemical constituents of Baiqin Qinghuo Keli by UPLC-TOF-MS' CENTRAL SOUTH PHARMACY vol. 9, no. 3, March 2011, pages 166 - 169, XP008179333
- GAO, HONG: 'Preliminary investigation for prescription injection of Radix codonopsis and Radix astragali' CHINESE MASTER'S THESES FULL-TEXT DATABASE no. 1, 15 January 2007, pages 47 - 56, XP055255079
- LI, XIANG ET AL.: 'Study on HPLC-MS total ionic chromatographic fingerprints of Radix astragali' CHIN PHARM J. vol. 41, no. 22, November 2006, pages 1745 - 1747, XP055265043
- ZHANG, YONGHUI ET AL.: 'Study on HPLC fingerprint of Dangshen in Shenqifuzheng injection' JOURNAL OF CHINESE MEDICINAL MATERIALS vol. 29, no. 12, December 2006, pages 1355 - 1358, XP008179275
- YUAN, XUJIANG ET AL.: 'Effective composition purification in Shenqi Fuzheng Prescription by different technologies' CHINESE TRADITIONAL PATENT MEDICINE vol. 32, no. 9, September 2010, pages 1514 - 1518, XP055254470
- RUI, WEN ET AL.: 'Analysis of Astragalus membranaceus and Astragalus fried with honey by UPLC/Q-TOF-MS' JOURNAL OF GUANGDONG PHARMACEUTICAL UNIVERSITY vol. 28, no. 1, February 2012, pages 47 - 50, XP 055254471
- HU, MINGXUN ET AL.: 'Quality analysis of semi-cultivated Astragalus membranaceus var. mongholicus from Hunyuan county of Shanxi province' CHINESE TRADITIONAL AND HERBAL DRUGS vol. 43, no. 9, September 2012, pages 1829 - 1834, XP055254475
- LIU MENG-HUA ET AL: "Rapid separation and identification of multiple constituents in traditional Chinese medicine formula Shenqi Fuzheng Injection by ultra-fast liquid chromatography combined with quadrupole-time-of-flight mass spectrometry", JOURNAL OF PHARMACEUTICAL AND BIOCHEMICAL ANALYSIS, ELSEVIER B.V, AMSTERDAM, NL, vol. 74, 26 October 2012 (2012-10-26), pages 141-155, XP028961182, ISSN: 0731-7085, DOI: 10.1016/J.JPBA.2012.10.024

## Description

### FIELD OF THE INVENTION

The present invention belongs to the field of drug testing, particularly relates to a method for establishing a Shenqi Fuzheng injection fingerprint profile.

### BACKGROUND OF THE INVENTION

Quality control of traditional Chinese herbal compound is one of the critical problems restricting development in modernization of traditional Chinese medicine. The basic theory of traditional Chinese medicine emphasizes the overall effect of the medicines and attaches importance to the synergistic effects on efficacy. Taking one or two effective components in Chinese medicines as qualitative and quantitative indexes is far from effectively controlling and assessing the quality of Chinese medicines, more difficult to reflect the safety and effectiveness of Chinese medicines. Traditional Chinese herbal compound is a compound preparation, which combines more than two kinds of Chinese medicinal herbs to be used in disease treatment, whose quality control is more difficult than single Chinese medicinal herb. In recent years, Chinese medicine fingerprint profile and characteristic fingerprint profile are widely used in quality control, wherein the Chinese medicine characteristic fingerprint profile refers to a characteristic fingerprint profile made up by selecting several chromatographic peaks with good specificity or a combination of chromatographic peaks with good specificity from the Chinese medicine fingerprint profile, which can be used to monitor the quality of Chinese medicines by observing the presence or absence and changes of the characteristic fingerprint peaks. In Chinese Pharmacopoeia, the 2010 Edition, the characteristic fingerprint profile has been widely applied in quality control of intermediate components of various Chinese medicine.

Ultra high performance liquid chromatography (UHPLC) technology is a great breakthrough of the chromatography technique. It has advantages in ultra-high speed, ultra-high sensitivity and ultra-high resolution, and it achieves faster and more sensitive detection performance by using a smaller chromatographic column packing technology. It is widely used in pesticide residues and drug metabolism in foreign countries, and its application become more and more common in China.

Chromatography-mass spectrometry technology is an advanced analysis technique developing rapidly in recent years, wherein, after extensive application of the gas chromatography-mass spectrometry (GC-MS) technology, the liquid chromatography-mass spectrometry (LC-MS) technology is an another technique which is gradually recognized and accepted , but it is not yet widely applied because of expensive equipment. The ionization technology adopted in liquid chromatography-mass spectrometers can not only resolve the problems in detecting some ingredients without ultraviolet absorption, such as saponins, but also get a precise molecular weight of the ionized component, which provides data support for identification and confirmation of the component and the structure thereof.

Shenqi Fuzheng injection is a Chinese medicine infusion, with effect of benefiting qi for strengthening resistance, and is used for the treatment of lassitude, shortness of breath with no desire to speak, spontaneous sweating and vertigo caused by lung-spleen deficiency, and used as an adjuvant therapy for lung cancer and gastric cancer patients with the syndromes above. It is one of National Protection Varieties of Traditional Chinese Medicine, and the Protection Variety No. is: ZYB2072004073. Among quality standards for Shenqi Fuzheng injection, the *"Fingerprint Profile"* item saying detection by using high performance liquid chromatography-ultraviolet detector has some limitations, for example, the main component saponin has no ultraviolet absorption. The component saponin may be monitored by using high performance liquid chromatography-evaporative light scattering detector, but pre-treatments for samples are complicated and furthermore the analysis takes a long time. Therefore, the main components cannot be monitored comprehensively and quickly, and there needs to be improved.

Liu Meng-Hua et al. ("Rapid separation and identification of multiple constituents in traditional Chinese medicine formula Shenqi Fuzheng Injection by ultra-fast liquid chromatography combined with quadrupole-time-of-flight mass spectrometry", Journal of Pharmaceutical and Biomedical Analysis 74, pages 141 to 155, published 26 October 2012) discloses a device and method for the identification of multiple constituents of SFI using ultrafast liquid chromatography equipped with a LC-20AD-XR pump which operates up to 66 MPa, an Agilent Eclipse plus C18 (2.1 mm × 100 mm, 1.8(jm) column held at 25°C and a time of flight mass spectrometer (page 142, right hand column, paragraph 2.3 "UFLC-Q-TOF-MS/MS system and conditions"). This passage further discloses mobile phases composed of methanol, acetonitrile and formic acid aqueous solutions and the use of a multi-step gradient from minutes 0 to 40 at a flow rate of 0.3 mL/min and an injection volume of 5 µ l. However, this passage does not care whether the detection object is real or fake.

### SUMMARY OF THE INVENTION

The technical problem to be solved in the present invention is to remedy deficiencies in prior art, and the objective of the present invention is to provide a method for establishing Shenqi Fuzheng injection fingerprint profile. The fingerprint profile established by the method described in the present invention can be used as a standard fingerprint profile to be applied in identification of Shenqi Fuzheng injection.

The present invention achieves the above objective by use of the following technical solutions:
A method for identifying Shenqi Fuzheng injection fingerprint profile by ultra-high pressure liquid chromatography-mass spectrometry, comprising the following steps:
step (1): establishing a profile of the samples to be tested;
step (2): establishing a characteristic fingerprint profile of Shenqi Fuzheng injection as the standard fingerprint profile;
step (3): comparing the profile of the samples to be tested in step (1) with the standard fingerprint profile in step (2) to distinguish between real and fake; wherein, the standard fingerprint profile in step (2) comprises 18 characteristic peaks, and the retention time and the mass number of the 18 characteristic peaks are as follows:
   Peak 1: 7.1 min (471.2083), Peak 2: 7.5 min (491.1195), Peak 3: 8.1 min (441.1919), Peak 4: 8.6 min (309.1555), Peak 5: 9.2 min (187.0976), Peak 6: 9.9 min (441.1766), Peak 7: 10.9 min (593.1876), Peak 8: 11.3 min (507.1508), Peak 9: 11.7 min (463.1610), Peak 10: 12.7 min (991.5119), Peak 11: 13.4 min (991.5119), Peak 12: 13.7 min (829.4591), Peak 13: 14.4 min (871.4697), Peak 14: 14.8 min (871.4697), Peak 15: 15.1 min (871.4697), Peak 16: 15.5 min (913.4650), Peak 17: 15.9 min (913.4650), Peak 18: 16.3 min (913.4650);
   wherein Peak 2 and Peak 12 are calycosin glucoside and astragaloside IV, respectively;
   wherein the method further comprises taking the control astragaloside IV as a reference peak of the Shenqi Fuzheng injection characteristic fingerprint profile, and calculating the relative retention time of each characteristic peak, as follows:
      Peak 1: 0.52, Peak 2: 0.54, Peak 3: 0.59, Peak 4: 0.62, Peak 5: 0.66, Peak 6: 0.72, Peak 7: 0.79, Peak 8: 0.82, Peak 9: 0.85, Peak 10: 0.92, Peak 11: 0.97, Peak 12: 1.00, Peak 13: 1.04, Peak 14: 1.07, Peak 15: 1.10, Peak 16: 1.13, Peak 17: 1.16, Peak 18: 1.19;
   wherein, when comparing, the relative retention time fluctuates within ±5% of the above specified values, and the ratio of the area of the calycosin glucoside peak and the astragaloside IV peak to the area of the corresponding reference peak is 0.5-1.5.

Preferably, wherein, the profile of the samples to be tested in step (1) was established by the following method, which comprising testing the samples to be tested by ultra-high pressure liquid chromatography-mass spectrometer, wherein the method comprises the following steps:
(1) preparation of control solution: accurately weighing an appropriate amount of calycosin glucoside or astragaloside IV, and then adding methanol to prepare a solution containing 0.004 mg of calycosin glucoside per ml or 0.006 mg of astragaloside IV per ml, respectively;
(2) preparation of test sample solution: filtering the samples to be tested through a 0.22 µm microporous filter membrane;
(3) determination: accurately aspirating 5 µl of the control solution or the test sample solution, respectively, and then injecting the solutions into a ultra-high pressure liquid chromatography-mass spectrometer, conducting determination according to the following conditions to obtain the fingerprint profile;
   wherein the chromatographic conditions include the followings:
   chromatographic column: Agilent Zorbax Eclipse Plus C18, 2.1 mm × 100 mm, 1.8 µm;
   mobile phase: mobile phase A is 0.1% (v/v) formic acid aqueous solution, mobile phase B is 0.1% (v/v) formic acid acetonitrile solution;
   using gradient elution according to the following elution program, wherein the proportions of the mobile phases are all volume percentages:
      0-0.5 min, mobile phase A is 95%, mobile phase B is 5%;
      0.5-10 min, mobile phase A is 95%-75%, mobile phase B is 5%-25%;
      10-15 min, mobile phase A is 75%-45%, mobile phase B is 25%-55%;
      15-18 min, mobile phase A is 45%-0%, mobile phase B is 55%-100%;
      18-20 min, mobile phase A is 0%, mobile phase B is 100%.
      flow rate: 0.35 ml/min;
      column temperature: 40 °C;
      the ion source is an ESI source, and detection is operated in negative ion mode;
      atomized gas pressure: 35 psig;
      dry gas temperature: 350 °C;
      dry gas flow rate: 10 L/min;
      capillary voltage: 3,500 V;
      voltage at capillary exit: 135 V.

Preferably, wherein, the Shenqi Fuzheng injection characteristic fingerprint profile in step (2) was established by the following method, which comprises testing Shenqi Fuzheng injection by ultra-high pressure liquid chromatography-mass spectrometer, wherein the method comprises the following steps:
(1) preparation of control solution: accurately weighing an appropriate amount of calycosin glucoside or astragaloside IV, and then adding methanol to prepare a solution containing 0.004 mg of calycosin glucoside per ml or 0.006 mg of astragaloside IV per ml, respectively;
(2) preparation of test sample solutions: filtering Shenqi Fuzheng injection through a 0.22 µm microporous filter membrane;
(3) determination: accurately aspirating 5 µl of the control solution or the test sample solution, respectively, and then injecting the solutions into a ultra-high pressure liquid chromatography-mass spectrometer, conducting determination according to the following conditions to obtain the Shenqi Fuzheng injection fingerprint profile;
   wherein the chromatographic conditions include the followings:
   chromatographic column: Agilent Zorbax Eclipse Plus C18, 2.1 mm × 100 mm, 1.8 µm;
   mobile phase: mobile phase A is 0.1% (v/v) formic acid aqueous solution, mobile phase B is 0.1% (v/v) formic acid acetonitrile solution;
   using gradient elution according to the following elution program, wherein the proportions of the mobile phases are all volume percentages:
      0-0.5 min, mobile phase A is 95%, mobile phase B is 5%;
      0.5-10 min, mobile phase A is 95%-75%, mobile phase B is 5%-25%;
      10-15 min, mobile phase A is 75%-45%, mobile phase B is 25%-55%;
      15-18 min, mobile phase A is 45%-0%, mobile phase B is 55%-100%;
      18-20 min, mobile phase A is 0%, mobile phase B is 100%.
   flow rate: 0.35 ml/min;
   column temperature: 40 °C;
   the ion source is an ESI source, and detection is operated in negative ion mode;
   atomized gas pressure: 35 psig;
   dry gas temperature: 350 °C;
   dry gas flow rate: 10 L/min;
   capillary voltage: 3,500 V;
   voltage at capillary exit: 135 V;
(4) determination of common characteristic peaks to obtain the standard fingerprint profile: comparing multiple Shenqi Fuzheng injection fingerprint profiles obtained from the above method, picking out 18 common characteristic peaks as the standard fingerprint profile.

Compared with the prior art, the present invention has beneficial effects as follows:
In the quality standards for Shenqi Fuzheng injection, the *"Fingerprint Profile"* item says that Shenqi Fuzheng injection fingerprint profile is determined by high performance liquid chromatography-UV detection, which achieves the objective of monitoring the quality to a certain extent, but the main component saponins substantially has no absorption under ultraviolet; the *"Content Determination"* item says that the content of total saponins is determined by UV spectrophotometry with vanillin-glacial acetic acid, and the content of astragaloside IV is determinded by high performance liquid chromatography-evaporative light scattering detection, but these still cannot fully reflect the content of each saponin component. In the internal control in enterprise, the saponin component can be monitored by further using high performance liquid chromatography-evaporative light scattering detector to determine the fingerprint profile, but pre-treatments for samples are required additionally, and the analysis would take a long time.

Therefore, the technical standards for Shenqi Fuzheng injection characteristic fingerprint profile established by ultra high performance liquid chromatography-quadrupole time-of-flight mass spectrometry according to the method of the present invention can make it easier to monitor drug quality comprehensively, quickly, and effectively, depending on presence or absence and characteristics of common peaks in the characteristic fingerprint profile, so as to ensure stable, uniform and controllable qualities. The present invention has features in is advanced method and better stability and reproducibility.

### BRIEF DESCRIPTION OF THE DRAWINGS

Figure 1 illustrates a total ion chromatogram (TIC) of the Shenqi Fuzheng injection,
   wherein the arrows from left to right indicate the characteristic peaks 1 to 18, respectively, provided by the present invention;
Figure 2 illustrates an extracted ion chromatogram (EIC) of the Shenqi Fuzheng injection, wherein the arrows from left to right indicate the characteristic peaks 1 to 18, respectively;
Figure 3 illustrates an extracted ion chromatogram (EIC) of the control mixture, wherein Peaks 2 and 12 represent calycosin glucoside and astragaloside IV, sequentially.
Figure 4 is a graph illustrating the comparison of the Shenqi Fuzheng injection fingerprint profile of the present invention with a counterfeit, wherein "1" showing the certified Shenqi Fuzheng injection, "2" showing the counterfeit (presumed as Danshen Injection), and "3" showing a Danshen infusion solution.

### DETAILED DESCRIPTION OF THE EMBODIMENTS

The technical solutions of the present invention will be further described in details in combination with specific examples.

### Example 1

1. Instrument and test drug
1.1 Instruments: Agilent LC/MSD (1290UHPLC dual-gradient pump, built-in vacuum degasser, 100-bit automatic sampler, intelligent column oven, high-precision quadrupole tandem time-of-flight mass spectrometer system); Chromatographic column: Agilent Zorbax Eclipse Plus C18 (2.1 mm ×100 mm, 1.8 µm).
1.2 Test drug: Shenqi Fuzheng injection, provided by Livzon Group Limin Pharmaceutical Factory. Reagents acetonitrile and formic acid used in the experiments were both chromatographically pure, and the water was ultrapure water.

2. Method and result
2.1 Preparation of test sample solution: Shenqi Fuzheng injection was filtered through a 0.22 µm microporous filter membrane.
2.2 Preparation of mixed control solution: An appropriate amount of calycosin glucoside and astragaloside IV was accurately weighed, and methanol was then added to prepare a solution containing 0.004 mg of calycosin glucoside and 0.006 mg of astragaloside IV per ml, respectively.
2.3 Chromatographic conditions: Chromatographic column was Agilent Zorbax Eclipse Plus C18, 2.1 mm × 100 mm, 1.8 µm; Mobile phases were 0.1% formic acid aqueous solution (A) and 0.1% formic acid acetonitrile solution (B); Column temperature was 40 °C; Injection volume was 5µl; A gradient elution program as shown in Table 2 was used:

**Table 2 Gradient Elution Program**

| Time (min) | Mobile Phase A(%) | Mobile Phase B (%) |
|---|---|---|
| 0-0.5 | 95 | 5 |
| 0.5-10 | 95 → 75 | 5 → 25 |
| 10-15 | 75 → 45 | 25 → 55 |
| 15-18 | 45 → 0 | 55 → 100 |
| 18-20 | 0 | 100 |

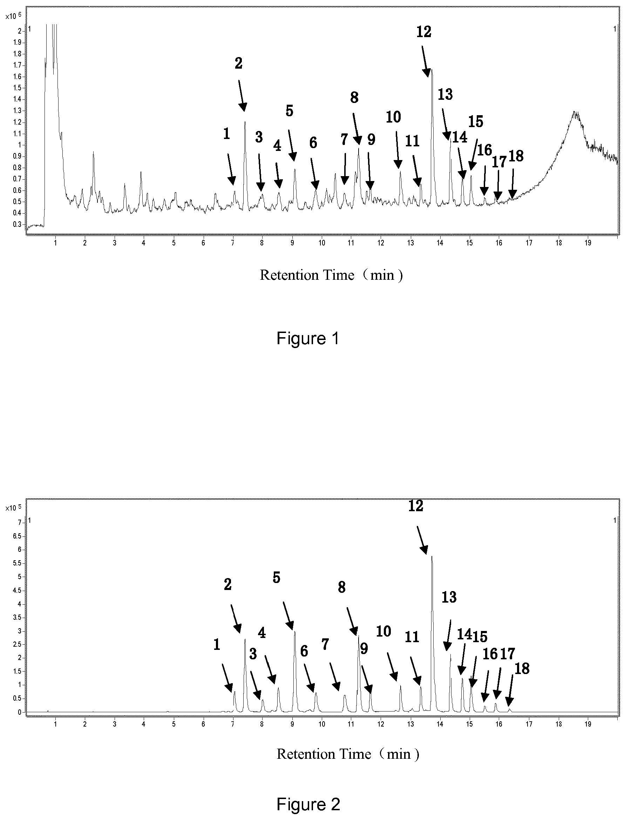

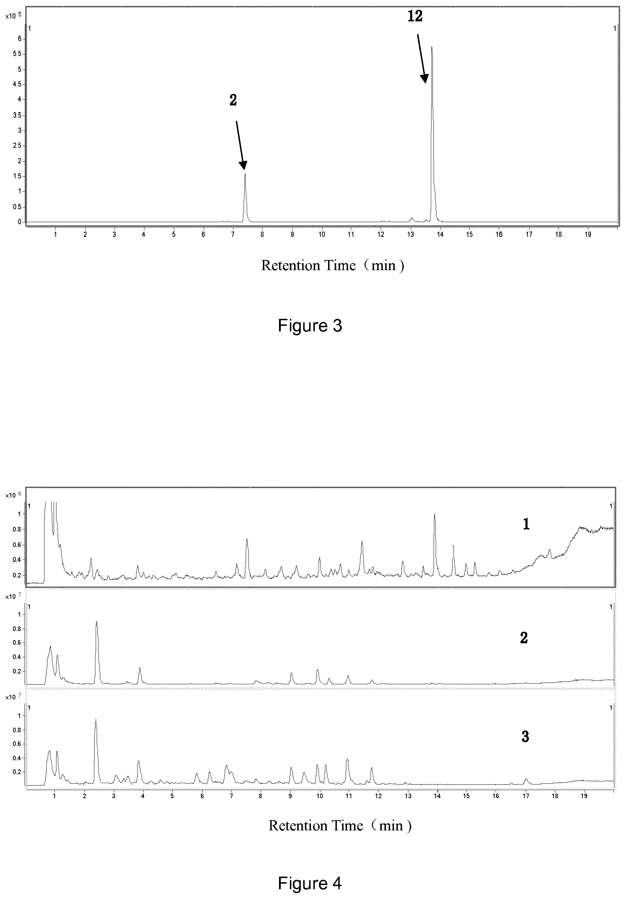

2.4 Mass spectrometry conditions: The ion source was an ESI source, detection was operated in negative ion mode; Atomized gas pressure: 35 psig (1 psig = 69 hPa); Dry gas temperature: 350 °C; Dry gas flow rate:10 L/min; Vcap Capillary voltage: 3,500 V; Voltage at capillary exit: 135 V.
2.5 Determination method 5 µl of the control solution or the test sample solution was accurately aspirated, respectively, then injected into the liquid chromatography-mass spectrometer, respectively, determination was conducted, recording the spectra for 20 minutes.
2.6 Determination of common characteristic peaks
The common characteristic peaks were sorted out by comparing the total ion chromatograms (TIC) of 100 batches of Shenqi Fuzheng injection, see details in Figure 1; the extracted ion chromatogram (EIC) was obtained by using the ion mass number of these common characteristic peaks, see details in Figure 2 (specifically, a series of target ions were extracted from Figure 1 by using the qualitative analysis software in the data analysis software Masshunter and utilizing the function of ion extraction, and thereby obtaining Figure 2); then the retention time (Rt) of each common characteristic peak was marked, so as to obtain the Shenqi Fuzheng injection characteristic fingerprint profile. There were 18 common characteristic peaks, with a retention time (Rt) and a mess number as follows: 7.1 min (471.2083), 7.5 min (491.1195), 8.1 min (441.1919), 8.6 min (309.1555), 9.2 min (187.0976), 9.9 min (441.1766), 10.9 min (593.1876), 11.3 min (507.1508), 11.7 min (463.1610), 12.7 min (991.5119), 13.4 min (991.5119), 13.7 min (829.4591), 14.4 min (871.4697), 14.8 min (871.4697), 15.1 min (871.4697), 15.5 min (913.4650), 15.9 min (913.4650), 16.3 min (913.4650), wherein the chromatographic peaks with a Rt of 7.5 min and 13.7 min were verified as calycosin glucoside and astragaloside IV respectively, see details in Figure 3; Peak S was a peak corresponding to an astragaloside IV reference peak, the retention time of each characteristic peaks was calculated, wherein the retention time should fluctuate within ±5% of the specified values, which were listed in an order of 0.52, 0.54, 0.59, 0.62, 0.66, 0.72, 0.79, 0.82, 0.85, 0.92, 0.97, 1.00, 1.04, 1.07, 1.10, 1.13, 1.16, 1.19; the ratio of the peak area of calycosin glucoside and astragaloside IV to the peak area of their corresponding reference should be within 0.5-1.5.
2.7 Precision assay: The test sample solution from the same Shenqi Fuzheng injection was injected for continuously 6 times, followed by extracting the characteristic peaks and marking the retention time. Peak 12 of astragaloside IV was taken as Peak S, and then the relative retention time of each of other characteristic peaks were calculated. The results showed that the RSD values of the relative retention time of each characteristic peak was all less than 1%, and the precision of the instrument was excellent. The results of the precision assay are shown in Table 3:

**Table 3 Results of Precision Assay**

| | 1 | 2 | 3 | 4 | 5 | 6 | RSD (%) |
|---|---|---|---|---|---|---|---|
| 1 | 0.517 | 0.517 | 0.517 | 0.516 | 0.516 | 0.517 | 0.14 |
| 2 | 0.542 | 0.542 | 0.542 | 0.541 | 0.541 | 0.542 | 0.09 |
| 3 | 0.586 | 0.586 | 0.586 | 0.585 | 0.585 | 0.586 | 0.08 |
| 4 | 0.623 | 0.624 | 0.624 | 0.623 | 0.623 | 0.624 | 0.08 |
| 5 | 0.661 | 0.661 | 0.661 | 0.661 | 0.661 | 0.661 | 0.04 |
| 6 | 0.716 | 0.715 | 0.716 | 0.715 | 0.716 | 0.716 | 0.06 |
| 7 | 0.786 | 0.786 | 0.787 | 0.787 | 0.788 | 0.787 | 0.08 |
| 8 | 0.820 | 0.819 | 0.820 | 0.820 | 0.820 | 0.821 | 0.08 |
| 9 | 0.847 | 0.847 | 0.848 | 0.848 | 0.849 | 0.848 | 0.06 |
| 10 | 0.923 | 0.922 | 0.923 | 0.922 | 0.923 | 0.923 | 0.01 |
| 11 | 0.972 | 0.972 | 0.972 | 0.972 | 0.972 | 0.972 | 0.02 |
| S | 1.000 | 1.000 | 1.000 | 1.000 | 1.000 | 1.000 | 0.00 |
| 13 | 1.044 | 1.044 | 1.044 | 1.045 | 1.044 | 1.044 | 0.02 |
| 14 | 1.074 | 1.074 | 1.074 | 1.074 | 1.074 | 1.074 | 0.02 |
| 15 | 1.096 | 1.096 | 1.096 | 1.096 | 1.096 | 1.096 | 0.01 |
| 16 | 1.129 | 1.129 | 1.128 | 1.129 | 1.129 | 1.129 | 0.03 |
| 17 | 1.155 | 1.155 | 1.155 | 1.156 | 1.156 | 1.155 | 0.02 |
| 18 | 1.189 | 1.189 | 1.189 | 1.189 | 1.189 | 1.189 | 0.02 |

2.8 Stability assay: The test sample solution from the same Shenqi Fuzheng injection was injected at 0 hour, at 1 hour, at 2 hours, at 4 hours, at 8 hours, and at 12 hours, respectively, followed by extracting the characteristic peaks and marking the retention time. Peak 12 of astragaloside IV was taken as Peak S, and then the relative retention time of each of other characteristic peaks was calculated. The results showed that the RSD values of the relative retention time of each characteristic peak was all less than 1%, and the test sample solution remained stable within 12 hours during standing. The results of the stability assay are shown in Table 4:

**Table 4 Results of Stability Assay**

| | 0 | 1 | 2 | 4 | 8 | 12 | RSD (%) |
|---|---|---|---|---|---|---|---|
| 1 | 0.517 | 0.516 | 0.516 | 0.517 | 0.517 | 0.517 | 0.15 |
| 2 | 0.542 | 0.541 | 0.541 | 0.542 | 0.541 | 0.543 | 0.11 |
| 3 | 0.586 | 0.586 | 0.585 | 0.586 | 0.586 | 0.587 | 0.09 |
| 4 | 0.624 | 0.623 | 0.623 | 0.624 | 0.623 | 0.624 | 0.09 |
| 5 | 0.662 | 0.662 | 0.661 | 0.662 | 0.661 | 0.662 | 0.05 |
| 6 | 0.716 | 0.716 | 0.715 | 0.716 | 0.715 | 0.716 | 0.06 |
| 7 | 0.787 | 0.786 | 0.786 | 0.788 | 0.787 | 0.786 | 0.07 |
| 8 | 0.820 | 0.820 | 0.819 | 0.820 | 0.820 | 0.821 | 0.04 |
| 9 | 0.848 | 0.848 | 0.847 | 0.848 | 0.848 | 0.847 | 0.04 |
| 10 | 0.922 | 0.922 | 0.923 | 0.923 | 0.922 | 0.923 | 0.02 |
| 11 | 0.972 | 0.972 | 0.972 | 0.972 | 0.972 | 0.972 | 0.01 |
| S | 1.000 | 1.000 | 1.000 | 1.000 | 1.000 | 1.000 | 0.00 |
| 13 | 1.044 | 1.044 | 1.044 | 1.044 | 1.044 | 1.045 | 0.01 |
| 14 | 1.074 | 1.074 | 1.074 | 1.074 | 1.074 | 1.074 | 0.01 |
| 15 | 1.096 | 1.096 | 1.096 | 1.096 | 1.096 | 1.096 | 0.02 |
| 16 | 1.129 | 1.129 | 1.129 | 1.129 | 1.129 | 1.129 | 0.02 |
| 17 | 1.155 | 1.156 | 1.156 | 1.155 | 1.156 | 1.156 | 0.02 |
| 18 | 1.189 | 1.190 | 1.189 | 1.189 | 1.189 | 1.190 | 0.04 |

2.9 Repeatability assay: Six Shenqi Fuzheng injection of the same batch were taken, prepared according to the method of preparing the test sample solution, and then injected, respectively, followed by extracting the characteristic peaks and marking the retention time. Peak 12 of astragaloside IV was taken as peak S, and then the relative retention time of each of other characteristic peaks was calculated, The results showed that the RSD values of the relative retention time of each characteristic peak was all less than 1%, and the method has good repeatability. The results of the repeatability assay are shown in Table 5:

**Table 5 Results of Repeatability Test**

| | 1 | 2 | 3 | 4 | 5 | 6 | RSD (%) |
|---|---|---|---|---|---|---|---|
| 1 | 0.512 | 0.511 | 0.511 | 0.512 | 0.511 | 0.517 | 0.45 |
| 2 | 0.537 | 0.536 | 0.536 | 0.536 | 0.536 | 0.542 | 0.45 |
| 3 | 0.581 | 0.581 | 0.580 | 0.580 | 0.581 | 0.586 | 0.40 |
| 4 | 0.619 | 0.619 | 0.618 | 0.619 | 0.618 | 0.624 | 0.38 |
| 5 | 0.655 | 0.655 | 0.655 | 0.655 | 0.655 | 0.661 | 0.37 |
| 6 | 0.711 | 0.710 | 0.710 | 0.710 | 0.709 | 0.716 | 0.36 |
| 7 | 0.782 | 0.782 | 0.781 | 0.782 | 0.781 | 0.786 | 0.25 |
| 8 | 0.815 | 0.815 | 0.815 | 0.815 | 0.815 | 0.820 | 0.25 |
| 9 | 0.843 | 0.844 | 0.844 | 0.844 | 0.844 | 0.848 | 0.18 |
| 10 | 0.922 | 0.922 | 0.922 | 0.922 | 0.922 | 0.923 | 0.03 |
| 11 | 0.972 | 0.972 | 0.972 | 0.972 | 0.972 | 0.972 | 0.01 |
| S | 1.000 | 1.000 | 1.000 | 1.000 | 1.000 | 1.000 | 0.00 |
| 13 | 1.045 | 1.044 | 1.045 | 1.044 | 1.044 | 1.044 | 0.02 |
| 14 | 1.074 | 1.075 | 1.075 | 1.075 | 1.074 | 1.074 | 0.03 |
| 15 | 1.096 | 1.097 | 1.097 | 1.096 | 1.096 | 1.096 | 0.04 |
| 16 | 1.129 | 1.129 | 1.130 | 1.129 | 1.128 | 1.129 | 0.05 |
| 17 | 1.156 | 1.156 | 1.156 | 1.156 | 1.155 | 1.155 | 0.04 |
| 18 | 1.190 | 1.190 | 1.190 | 1.190 | 1.190 | 1.189 | 0.04 |

2.10 Intermediate precision: Shenqi Fuzheng injections taken from the same batch were assayed according to the method described above, respectively, except for under the variable factors such as on different dates and by different analysts.
2.10.1 Different analysis dates: Shenqi Fuzheng injections taken from the same batch were prepared according to the method of preparing test sample solution on different dates, respectively, and then three of the test sample solution were injected in parallel, followed by extracting the characteristic peaks and marking the retention time. Peak 12 of astragaloside IV was taken as Peak S, and then the relative retention time of each of other characteristic peaks. The results showed that the RSD values of the relative retention time of each characteristic peak was all less than 1 %, as shown in Table 6:

**Table 6 Results of analysis on different dates**

| | Date 1 | | | Date 2 | | | RSD (%) |
|---|---|---|---|---|---|---|---|
| 1 | 0.517 | 0.518 | 0.517 | 0.516 | 0.516 | 0.517 | 0.19 |
| 2 | 0.541 | 0.541 | 0.542 | 0.542 | 0.541 | 0.541 | 0.08 |
| 3 | 0.585 | 0.586 | 0.585 | 0.585 | 0.585 | 0.586 | 0.03 |
| 4 | 0.623 | 0.624 | 0.623 | 0.623 | 0.623 | 0.623 | 0.07 |
| 5 | 0.661 | 0.661 | 0.661 | 0.661 | 0.661 | 0.661 | 0.05 |
| 6 | 0.715 | 0.715 | 0.715 | 0.715 | 0.715 | 0.715 | 0.01 |
| 7 | 0.786 | 0.786 | 0.787 | 0.786 | 0.786 | 0.786 | 0.03 |
| 8 | 0.819 | 0.820 | 0.820 | 0.820 | 0.820 | 0.819 | 0.02 |
| 9 | 0.847 | 0.847 | 0.848 | 0.848 | 0.848 | 0.847 | 0.03 |
| 10 | 0.923 | 0.923 | 0.923 | 0.923 | 0.922 | 0.922 | 0.01 |
| 11 | 0.972 | 0.972 | 0.973 | 0.972 | 0.972 | 0.972 | 0.02 |
| S | 1.000 | 1.000 | 1.000 | 1.000 | 1.000 | 1.000 | 0.00 |
| 13 | 1.044 | 1.044 | 1.044 | 1.045 | 1.044 | 1.044 | 0.02 |
| 14 | 1.074 | 1.074 | 1.074 | 1.074 | 1.074 | 1.074 | 0.01 |
| 15 | 1.096 | 1.097 | 1.096 | 1.096 | 1.096 | 1.096 | 0.03 |
| 16 | 1.128 | 1.129 | 1.129 | 1.129 | 1.128 | 1.128 | 0.03 |
| 17 | 1.155 | 1.156 | 1.155 | 1.156 | 1.156 | 1.155 | 0.03 |
| 18 | 1.189 | 1.189 | 1.190 | 1.189 | 1.189 | 1.188 | 0.04 |

2.10.2 Different analysts: Shenqi Fuzheng injections taken from the same batch were prepared according to the method of preparing test sample solution by different analysts, respectively, and three of the test sample solution were injected in parallel, followed by extracting the characteristic peaks and marking the retention time. Peak 12 of astragaloside IV was taken as Peak S, and then the relative retention time of each of other characteristic peaks was calculated. The results showed that the RSD values of the relative retention time of each characteristic peak was all less than 1 %, as shown in Table 7:

**Table 7 Results of analysis by different analysts**

| | Analyst 1 | | | Analyst 2 | | | RSD (%) |
|---|---|---|---|---|---|---|---|
| 1 | 0.516 | 0.518 | 0.519 | 0.519 | 0.519 | 0.519 | 0.18 |
| 2 | 0.542 | 0.544 | 0.544 | 0.544 | 0.543 | 0.544 | 0.16 |
| 3 | 0.586 | 0.588 | 0.588 | 0.589 | 0.586 | 0.587 | 0.18 |
| 4 | 0.623 | 0.625 | 0.625 | 0.626 | 0.624 | 0.625 | 0.16 |
| 5 | 0.661 | 0.663 | 0.663 | 0.663 | 0.661 | 0.663 | 0.17 |
| 6 | 0.714 | 0.716 | 0.717 | 0.717 | 0.714 | 0.716 | 0.19 |
| 7 | 0.786 | 0.788 | 0.789 | 0.789 | 0.785 | 0.788 | 0.19 |
| 8 | 0.819 | 0.821 | 0.822 | 0.822 | 0.819 | 0.821 | 0.15 |
| 9 | 0.848 | 0.848 | 0.849 | 0.849 | 0.847 | 0.848 | 0.07 |
| 10 | 0.922 | 0.922 | 0.923 | 0.923 | 0.922 | 0.923 | 0.03 |
| 11 | 0.972 | 0.972 | 0.972 | 0.972 | 0.972 | 0.972 | 0.03 |
| S | 1.000 | 1.000 | 1.000 | 1.000 | 1.000 | 1.000 | 0.00 |
| 13 | 1.044 | 1.044 | 1.044 | 1.044 | 1.044 | 1.044 | 0.02 |
| 14 | 1.074 | 1.074 | 1.074 | 1.074 | 1.074 | 1.074 | 0.01 |
| 15 | 1.096 | 1.095 | 1.095 | 1.095 | 1.096 | 1.096 | 0.02 |
| 16 | 1.128 | 1.129 | 1.129 | 1.128 | 1.128 | 1.128 | 0.02 |
| 17 | 1.155 | 1.155 | 1.155 | 1.155 | 1.155 | 1.156 | 0.03 |
| 18 | 1.189 | 1.188 | 1.188 | 1.188 | 1.189 | 1.189 | 0.04 |

### Example 2 Identification of Shenqi Fuzheng injection

In recent years, as the use of Shenqi Fuzheng injection is increasing in clinical, some criminals are motivated by economic interest and counterfeit Shenqi Fuzheng injection with other varieties for sale to make huge profits, which results in a great negative impact on the brands for Shenqi Fuzheng injection, and has caused significant economic loss to these enterprises which produce and sell Shenqi Fuzheng injections legally. These counterfeit Shenqi Fuzheng injections have almost the same appearance as the real ones, so it is hard to distinguish the real from the fake.

In this example, the method described in Example 1 was adopted to test the certified Shenqi Fuzheng injection provided by Livzon Group Limin Pharmaceutical Factory), a suspected sample and a Danshen injection, so as to establish the corresponding fingerprint profiles. The results were shown in Figure 4. It can be seen that the fingerprint profile of the suspected sample is completely different from that of the certified Shenqi Fuzheng injection, the components of the counterfeit were inferred by using the precise molecular weight provided by mass spectrometry. It was substantially confirmed that the components of the counterfeit were derived from Danshen.

Thus, the Shenqi Fuzheng injection fingerprint profile established by ultra high pressure liquid chromatography-mass spectrometer (UHPLC-MS) can be used to identify the authenticity of a Shenqi Fuzheng injection in a fast and accurate manner, and can also be used to analyze the counterfeit qualitatively and substantially confirm the source. If there is any counterfeit Shenqi Fuzheng injection with DanShen injection by criminals, it can be identified according to the method described above, i.e. comparing the fingerprint profile of the injection with that of the certified Shenqi Fuzheng injection, inferring the components of the counterfeit by using the precise molecular weight provided by mass spectrometry, substantially confirming the component source of the counterfeit.

Therefore, application of the Shenqi Fuzheng injection UHPLC-MS fingerprint profile can avoid counterfeiting, and ensure normal production and good circulation of the Shenqi Fuzheng injection so as to protect legitimate rights and interests of manufactures.

The method provided by the present invention for establishing Shenqi Fuzheng injection fingerprint profile was described in details hereinbefore. The principles and embodiments of the present invention are described herein with reference to some specific examples, however, the examples described above are only intended to help understand the method and the core idea of the present invention. It should be noted that, for those skilled in the art, a number of improvements and modifications can be introduced to the present invention, without departing from the principles of the present invention, and these improvements and modifications shall fall into the scope defined by the appended claims.

## Claims

1. A method for identifying a Shenqi Fuzheng injection fingerprint profile by ultra-high pressure liquid chromatography-mass spectrometry, comprising the following steps:
step (1): establishing a profile of the samples to be tested;
step (2): establishing a characteristic fingerprint profile of Shenqi Fuzheng injection as the standard fingerprint profile;
step (3): comparing the profile of the samples to be tested in step (1) with the standard fingerprint profile in step (2) to distinguish between real and fake;
wherein, the standard fingerprint profile in step (2) comprises 18 characteristic peaks, and the retention time and the mass number of the 18 characteristic peaks are as follows:
Peak 1: 7.1 min (471.2083), Peak 2: 7.5 min (491.1195), Peak 3: 8.1 min (441.1919), Peak 4: 8.6 min (309.1555), Peak 5: 9.2 min (187.0976), Peak 6: 9.9 min (441.1766), Peak 7: 10.9 min (593.1876), Peak 8: 11.3 min (507.1508), Peak 9: 11.7 min (463.1610), Peak 10: 12.7 min (991.5119), Peak 11: 13.4 min (991.5119), Peak 12: 13.7 min (829.4591), Peak 13: 14.4 min (871.4697), Peak 14: 14.8 min (871.4697), Peak 15: 15.1 min (871.4697), Peak 16: 15.5 min (913.4650), Peak 17: 15.9 min (913.4650), Peak 18: 16.3 min (913.4650);
wherein Peak 2 and Peak 12 are calycosin glucoside and astragaloside IV, respectively;
wherein the method further comprises taking the control astragaloside IV as a reference peak of the Shenqi Fuzheng injection characteristic fingerprint profile, and calculating the relative retention time of each characteristic peak, as follows:
Peak 1: 0.52, Peak 2: 0.54, Peak 3: 0.59, Peak 4: 0.62, Peak 5: 0.66, Peak 6: 0.72, Peak 7: 0.79, Peak 8: 0.82, Peak 9: 0.85, Peak 10: 0.92, Peak 11: 0.97, Peak 12: 1.00, Peak 13: 1.04, Peak 14: 1.07, Peak 15: 1.10, Peak 16: 1.13, Peak 17: 1.16, Peak 18: 1.19;
wherein, when comparing, the relative retention time fluctuates within ±5% of the above specified values, and the ratio of the area of the calycosin glucoside peak and the astragaloside IV peak to the area of the corresponding reference peak is 0.5-1.5.

2. The method according to claim 1, wherein, the profile of the samples to be tested in step (1) was established by the following method, which comprising testing the samples to be tested by ultra-high pressure liquid chromatography-mass spectrometer, wherein the method comprises the following steps:
(1) preparation of control solution: accurately weighing an appropriate amount of calycosin glucoside or astragaloside IV, and then adding methanol to prepare a solution containing 0.004 mg of calycosin glucoside per ml or 0.006 mg of astragaloside IV per ml, respectively;
(2) preparation of test sample solution: filtering the samples to be tested through a 0.22 µm microporous filter membrane;
(3) determination: accurately aspirating 5 µl of the control solution or the test sample solution, respectively, and then injecting the solutions into a ultra-high pressure liquid chromatography-mass spectrometer, conducting determination according to the following conditions to obtain the fingerprint profile;
wherein the chromatographic conditions include the followings:
chromatographic column: Agilent Zorbax Eclipse Plus C18, 2.1 mm × 100 mm, 1.8 µm;
mobile phase: mobile phase A is 0.1% (v/v) formic acid aqueous solution, mobile phase B is 0.1% (v/v) formic acid acetonitrile solution;
using gradient elution according to the following elution program, wherein the proportions of the mobile phases are all volume percentages:
0-0.5 min, mobile phase A is 95%, mobile phase B is 5%;
0.5-10 min, mobile phase A is 95%-75%, mobile phase B is 5%-25%;
10-15 min, mobile phase A is 75%-45%, mobile phase B is 25%-55%;
15-18 min, mobile phase A is 45%-0%, mobile phase B is 55%-100%;
18-20 min, mobile phase A is 0%, mobile phase B is 100%.
flow rate: 0.35 ml/min;
column temperature: 40 °C;
the ion source is an ESI source, and detection is operated in negative ion mode;
atomized gas pressure: 35 psig (1 psig = 69 hPa);
dry gas temperature: 350 °C;
dry gas flow rate: 10 L/min;
capillary voltage: 3,500 V;
voltage at capillary exit: 135 V.

3. The method according to claim 1, wherein, the Shenqi Fuzheng injection characteristic fingerprint profile in step (2) was established by the following method, which comprises testing Shenqi Fuzheng injection by ultra-high pressure liquid chromatography-mass spectrometer, wherein the method comprises the following steps:
(1) preparation of control solution: accurately weighing an appropriate amount of calycosin glucoside or astragaloside IV, and then adding methanol to prepare a solution containing 0.004 mg of calycosin glucoside per ml or 0.006 mg of astragaloside IV per ml, respectively;
(2) preparation of test sample solutions: filtering Shenqi Fuzheng injection through a 0.22 µm microporous filter membrane;
(3) determination: accurately aspirating 5 µl of the control solution or the test sample solution, respectively, and then injecting the solutions into a ultra-high pressure liquid chromatography-mass spectrometer, conducting determination according to the following conditions to obtain the Shenqi Fuzheng injection fingerprint profile;
wherein the chromatographic conditions include the followings:
chromatographic column: Agilent Zorbax Eclipse Plus C18, 2.1 mm × 100 mm, 1.8 µm;
mobile phase: mobile phase A is 0.1% (v/v) formic acid aqueous solution, mobile phase B is 0.1% (v/v) formic acid acetonitrile solution;
using gradient elution according to the following elution program, wherein the proportions of the mobile phases are all volume percentages:
0-0.5 min, mobile phase A is 95%, mobile phase B is 5%;
0.5-10 min, mobile phase A is 95%-75%, mobile phase B is 5%-25%;
10-15 min, mobile phase A is 75%-45%, mobile phase B is 25%-55%;
15-18 min, mobile phase A is 45%-0%, mobile phase B is 55%-100%;
18-20 min, mobile phase A is 0%, mobile phase B is 100%.
flow rate: 0.35 ml/min;
column temperature: 40 °C;
the ion source is an ESI source, and detection is operated in negative ion mode;
atomized gas pressure: 35 psig;
dry gas temperature: 350 °C;
dry gas flow rate: 10 L/min;
capillary voltage: 3,500 V;
voltage at capillary exit: 135 V;
(4) determination of common characteristic peaks to obtain the standard fingerprint profile: comparing multiple Shenqi Fuzheng injection fingerprint profiles obtained from the above method, picking out 18 common characteristic peaks as the standard fingerprint profile.

## Patentansprüche

1. Ein Verfahren zur Identifizierung eines Shenqi Fuzheng Injektions-Fingerabdruckprofils mittels Ultrahochdruck-Flüssigkeitschromatographie
- Massenspektrometer, das die folgenden Schritte umfasst:
Schritt (1): Erstellung eines Profils der zu untersuchenden Proben;
Schritt (2): Erstellung eines charakteristischen Fingerabdruckprofils der Shenqi Fuzheng-Injektion als Standard-Fingerabdruckprofil;
Schritt (3): Vergleich des Profils der zu testenden Proben in Schritt (1) mit dem Standard-Fingerabdruckprofil in Schritt (2), um zwischen echt und gefälscht zu unterscheiden; wobei das Standard-Fingerabdruckprofil in Schritt (2) 18 charakteristische Peaks umfasst und die Retentionszeit und die Massenzahl der 18 charakteristischen Peaks wie folgt sind:
Peak 1: 7,1 min (471.2083), Peak 2: 7,5 min (491.1195), Peak 3: 8,1 min (441,1919), Peak 4: 8,6 min (309.1555), Peak 5: 9,2 min (187.0976), Peak 6: 9,9 min (441.1766), Peak 7: 10,9 min (593.1876), Peak 8: 11,3 min (507.1508), Peak 9: 11,7 min (463.1610), Peak 10: 12,7 min (991.5119), Peak 11: 13,4 min (991.5119), Peak 12: 13,7 min (829.4591), Peak 13: 14,4 min (871.4697), Peak 14: 14,8 min (871.4697), Peak 15: 15,1 min (871.4697), Peak 16: 15,5 min (913.4650), Peak 17: 15,9 min (913.4650), Peak 18: 16,3 min (913.4650);
wobei Peak 2 und Peak 12 Calycosin-Glucosid bzw. Astragalosid IV sind;
wobei das Verfahren ferner umfasst, dass das Kontroll Astragalosid IV als ein Referenzpeak des charakteristischen Fingerabdruckprofils der Shenqi Fuzheng-Injektion genommen wird und die relative Retentionszeit jedes charakteristischen Peaks wie folgt berechnet wird:
Peak 1: 0,52, Peak 2: 0,54, Peak 3: 0,59, Peak 4: 0,62, Peak 5: 0,66, Peak 6: 0,72, Peak 7: 0,79, Peak 8: 0,82, Peak 9: 0,85, Peak 10: 0,92, Peak 11: 0,97, Peak 12: 1,00, Peak 13: 1,04, Peak 14: 1,07, Peak 15: 1,10, Peak 16: 1,13, Peak 17: 1,16, Peak 18: 1,19;
wobei beim Vergleich die relative Retentionszeit innerhalb von ±5 % der oben angegebenen Werte schwankt und das Verhältnis der Fläche des Calycosin-Glucosid-Peaks und des Astragalosid-IV-Peaks zur Fläche des entsprechenden Referenzpeaks 0,5-1,5 beträgt.

2. Das Verfahren nach Anspruch 1, wobei das Profil der in Schritt (1) zu testenden Proben durch das folgende Verfahren erstellt wurde, das das Testen der zu testenden Proben mittels Ultrahochdruck-Flüssigkeitschromatographie-Massen spektrometer umfasst, wobei das Verfahren die folgenden Schritte umfasst:
(1) Herstellung der Kontrolllösung: Genaues Abwiegen einer geeigneten Menge von Calycosin-Glucosid oder Astragalosid IV und anschließende Zugabe von Methanol zur Herstellung einer Lösung, die 0,004 mg Calycosin-Glucosid pro ml bzw. 0,006 mg Astragalosid IV pro ml enthält;
(2) Herstellung der Testprobenlösung: Filtrieren der zu testenden Proben durch eine mikroporöse Filtermembran von 0,22 µm;
(3) Bestimmung: Genaue Entnahme von 5 µl der Kontrolllösung bzw. der Testprobenlösung und anschließende Injektion der Lösungen in ein Ultrahochdruck-Flüssigkeitschromatographie-Massenspektrometer, Durchführung der Bestimmung gemäß den folgenden Bedingungen, um das Fingerabdruckprofil zu erhalten;
wobei die chromatographischen Bedingungen die folgenden umfassen:
Chromatographiesäule: Agilent Zorbax Eclipse Plus C18, 2,1 mm × 100 mm, 1,8 µm;
mobile Phase: mobile Phase A ist 0,1% (v/v) wässrige Ameisensäurelösung, mobile Phase B ist 0,1% (v/v) Ameisensäure-Acetonitril-Lösung;
unter Verwendung einer Gradientenelution gemäß dem folgenden Elutionsprogramm, wobei die Anteile der mobilen Phasen alle Volumenprozentwerte sind:
0-0,5 min, mobile Phase A ist 95%, mobile Phase B ist 5%-25%;
0,5-10 min, mobile Phase A ist 95%-75%, mobile Phase B ist 5%-25%;
10-15 min, mobile Phase A ist 75%-45%, mobile Phase B ist 25%-55%;
15-18 min, mobile Phase A ist 45%-0%, mobile Phase B ist 55%-100%;
18-20 min, mobile Phase A ist 0%, mobile Phase B is 100%.
Durchflussrate: 0,35 ml/min;
Säulentemperatur: 40 °C;
die lonenquelle ist eine ESI-Quelle, und die Detektion erfolgt im Negativ-Ionen-Modus;
Zerstäubungsgasdruck: 35 psig (1 psig = 69 hPa);
Temperatur des Trockengases: 350 °C;
Durchflussrate des Trockengases: 10 L/min;
Kapillarspannung: 3.500 V;
Spannung am Kapillarausgang: 135 V.

3. Das Verfahren nach Anspruch 1, wobei das charakteristische Fingerabdruckprofil der Shenqi Fuzheng-Injektion in Schritt (2) durch das folgende Verfahren erstellt wurde, das das Testen der Shenqi Fuzheng-Injektion mittels Ultrahochdruck-Flüssigkeitschromatographie-Massenspektrometer umfasst, wobei das Verfahren die folgenden Schritte umfasst:
(1) Herstellung der Kontrolllösung: Genaues Abwiegen einer geeigneten Menge von Calycosin-Glucosid oder Astragalosid IV und anschließende Zugabe von Methanol zur Herstellung einer Lösung, die 0,004 mg Calycosin-Glucosid pro ml bzw. 0,006 mg Astragalosid IV pro ml enthält;
(2) Herstellung der Testprobenlösungen: Filtrieren der Shenqi Fuzheng-Injektion durch eine mikroporöse Filtermembran von 0,22 µm;
(3) Bestimmung: Genaue Entnahme von 5 µl der Kontrolllösung bzw. der Testprobenlösung und anschließende Injektion der Lösungen in ein Ultrahochdruck-Flüssigkeitschromatographie-Massenspektrometer, Durchführung der Bestimmung gemäß den folgenden Bedingungen, um das Fingerabdruckprofil der Shenqi Fuzheng-Injektion zu erhalten;
wobei die chromatographischen Bedingungen die folgenden umfassen:
Chromatographiesäule: Agilent Zorbax Eclipse Plus C18, 2,1 mm × 100 mm, 1,8 µm;
mobile Phase: mobile Phase A ist 0,1 % (v/v) wässrige Ameisensäurelösung, mobile Phase B ist 0,1 % (v/v) Ameisensäure-Acetonitril-Lösung;
unter Verwendung einer Gradientenelution gemäß dem folgenden Elutionsprogramm, wobei die Anteile der mobilen Phasen alle Volumenprozentwerte sind:
0-0,5 min, mobile Phase A ist 95%, mobile Phase B ist 5%;
0,5-10 min, mobile Phase A ist 95%-75%, mobile Phase B ist 5%-25%;
10-15 min, mobile Phase A ist 75%-45%, mobile Phase B ist 25%-55%;
15-18 min, mobile Phase A ist 45%-0%, mobile Phase B ist 55%-100%;
18-20 min, mobile Phase A ist 0%, mobile Phase B ist 100%.
Durchflussrate: 0,35 ml/min;
Säulentemperatur: 40 °C;
die lonenquelle ist eine ESI-Quelle, und die Detektion erfolgt im Negativ-Ionen-Modus;
Zerstäubungsgasdruck: 35 psig;
Temperatur des Trockengases: 350 °C;
Durchflussrate des Trockengases: 10 L/min;
Kapillarspannung: 3.500 V;
Spannung am Kapillarausgang: 135 V;
(4) Bestimmung gemeinsamer charakteristischer Peaks, um das Standard-Fingerabdruckprofil zu erhalten: Vergleich mehrerer Shenqi Fuzheng Injektions-Fingerabdruckprofile, die mit der oben genannten Methode erhalten wurden, Auswahl von 18 gemeinsamen charakteristischen Peaks als Standard-Fingerabdruckprofil.

## Revendications

1. Procédé d'identification d'un profil d'empreinte digitale d'injection de Shenqi Fuzheng par chromatographie liquide à ultra-haute pression-spectrométrie de masse, comportant les étapes suivantes:
étape (1): établissement d'un profil des échantillons à analyser;
étape (2): établissement d'un profil caractéristique d'empreinte digitale d'injection de Shenqi Fuzheng comme profil standard d'empreinte digitale;
étape (3): comparaison du profil des échantillons à analyser dans l'étape (1) au profil standard d'empreinte digitale dans l'étape (2) pour distinguer le vrai du faux;
dans lequel le profil standard d'empreinte digitale dans l'étape (2) comporte 18 pics caractéristiques, et le temps de rétention et le nombre de masse des 18 pics caractéristiques est comme suit:
pic 1: 7,1 min (471.2083), pic 2: 7,5 min (491.1195), pic 3: 8,1 min (441.1919), pic 4: 8,6 min (309.1555), pic 5: 9,2 min (187.0976), pic 6: 9,9 min (441.1766), pic 7: 10,9 min (593.1876), pic 8: 11,3 min (507.1508), pic 9: 11,7 min (463.1610), pic 10: 12,7 min (991.5119), pic 11: 13,4 min (991.5119), pic 12: 13,7 min (829.4591), pic 13: 14,4 min (871.4697), pic 14: 14,8 min (871.4697), pic 15: 15,1 min (871.4697), pic 16: 15,5 min (913.4650), pic 17: 15,9 min (913.4650), pic 18: 16,3 min (913.4650);
dans lequel le pic 2 et le pic 12 sont respectivement le glucoside de calycosine et l'astragaloside IV;
dans lequel le procédé consiste en outre à prendre l'astragaloside IV témoin comme pic de référence du profil caractéristique d'empreinte digitale d'injection de Shenqi Fuzheng, et à calculer le temps de rétention relatif de chaque pic caractéristique comme suit:
pic 1: 0,52, pic 2: 0,54, pic 3: 0,59, pic 4: 0,62, pic 5: 0,66, pic 6: 0,72, pic 7: 0,79, pic 8: 0,82, pic 9: 0,85, pic 10: 0,92, pic 11: 0,97, pic 12: 1,00, pic 13: 1,04, pic 14: 1,07, pic 15: 1,10, pic 16: 1,13, pic 17: 1,16, pic 18: 1,19;
dans lequel, en comparant, le temps de rétention relatif fluctue dans une fourchette de ± 5% des valeurs spécifiées ci-dessus, et le rapport entre la surface du pic du glucoside de calycosine et du pic d'astragaloside IV et la surface du pic de référence correspondant est de 0,5 à 1,5.

2. Procédé selon la revendication 1, dans lequel le profil des échantillons à analyser dans l'étape (1) a été établi par le procédé suivant comportant l'analyse des échantillons à analyser par un spectromètre de masse à chromatographie liquide à ultra-haute pression, dans lequel le procédé comporte les étapes suivantes:
(1) préparation de la solution témoin: peser avec précision une quantité appropriée de glucoside de calycosine ou d'astragaloside IV, et ensuite ajouter du méthanol pour préparer une solution contenant respectivement 0,004 mg de glycoside de calycosine par ml ou 0,006 mg d'astragaloside IV par ml;
(2) préparation d'une solution d'échantillon d'essai: filtrer les échantillons à analyser à travers une membrane filtrante microporeuse de 0,22 µm;
(3) détermination: aspirer avec précision 5 µl respectivement de la solution témoin ou de la solution d'échantillon d'essai, et ensuite injecter les solutions dans un spectromètre de masse à chromatographie liquide à ultra-haute pression effectuant la détermination selon les conditions suivantes pour obtenir le profil d'empreinte digitale;
dans lequel les conditions chromatographiques sont les suivantes:
colonne chromatographique: Agilent Zorbax Eclipse Plus C18, 2,1 mm × 100 mm, 1,8 µm;
phase mobile: phase mobile A est de 0,1% (v/v) de solution aqueuse d'acide formique, phase mobile B est de 0,1% (v/v) de solution acétonitrile d'acide formique;
en utilisant l'élution par gradient selon le programme suivant d'élution, dans lequel les proportions des phases mobiles sont toutes des pourcentages en volume:
0 à 0,5 min, phase mobile A est de 95%, phase mobile B est de 5%;
0,5 à 10 min, phase mobile A est de 95% à 75%, phase mobile B est de 5% à 25%;
10 à 15 min, phase mobile A est de 75% à 45%, phase mobile B est de 25% à 55%;
15 à18 min, phase mobile A est de 45% à 0%, phase mobile B est de 55% à 100%;
18 à 20 min, phase mobile A est de 0%, phase mobile B est de 100% débit: 0,35 ml/min;
température de la colonne: 40 °C;
la source d'ion est une source ESI et la détection fonctionne en mode ion négatif;
pression du gaz atomisé: 35 psig (1 psig = 69 hPa);
température du gaz sec: 350 °C;
débit du gaz sec: 10 L/min;
tension capillaire: 3.500 V.
tension à la sortie du capillaire: 135 V.

3. Procédé selon la revendication 1, dans lequel le profil caractéristique d'empreinte digitale d'injection de Shenqi Fuzheng dans l'étape (2) a été établi par le procédé suivant, qui comporte l'essai de l'injection de Shenqi Fuzheng par un spectromètre de masse à chromatographie liquide à ultra-haute pression, dans lequel le procédé comporte les étapes suivantes:
(1) préparation de la solution témoin: peser avec précision une quantité appropriée de glucoside de calycosine ou d'astragaloside IV, et ensuite ajouter du méthanol pour préparer une solution contenant respectivement 0,004 mg de glycoside de calycosine par ml ou 0,006 mg d'astragaloside IV par ml;
(2) préparation des solutions d'échantillon d'essai: filtrer l'injection de Shenqi Fuzheng à travers une membrane filtrante microporeuse de 0,22 µm;
(3) détermination: aspirer avec précision 5 µl respectivement de la solution témoin ou de la solution d'échantillon d'essai, et ensuite injecter les solutions dans un spectromètre de masse à chromatographie liquide à ultra-haute pression effectuant la détermination selon les conditions suivantes pour obtenir le profil d'empreinte digitale de l'injection de Shenqi Fuzheng;
dans lequel les conditions chromatographiques sont les suivantes:
colonne chromatographique: Agilent Zorbax Eclipse Plus C18, 2,1 mm × 100 mm, 1,8 µm;
phase mobile: phase mobile A est de 0,1% (v/v) de solution aqueuse d'acide formique, phase mobile B est de 0,1% (v/v) de solution acétonitrile d'acide formique;
en utilisant l'élution par gradient selon le programme suivant d'élution, dans lequel les proportions des phases mobiles sont toutes des pourcentages en volume:
0 à 0,5 min, phase mobile A est de 95%, phase mobile B est de 5%;
0,5 à 10 min, phase mobile A est de 95% à 75%, phase mobile B est de 5% à 25%;
10 à 15 min, phase mobile A est de 75% à 45%, phase mobile B est de 25% à 55%;
15 à18 min, phase mobile A est de 45% à 0%, phase mobile B est de 55% à 100%;
18 à 20 min, phase mobile A est de 0%, phase mobile B est de 100% débit: 0,35 ml/min;
température de la colonne: 40 °C;
la source d'ion est une source ESI et la détection fonctionne en mode ion négatif;
pression du gaz atomisé: 35 psig;
température du gaz sec: 350 °C;
débit du gaz sec: 10 L/min;
tension capillaire: 3.500 V;
tension à la sortie du capillaire: 135 V;
(4) détermination des pics caractéristiques communs pour obtenir le profil standard d'empreinte digitale: comparaison des profils multiples d'empreinte digitale d'injection de Shenqi Fuzheng obtenus par le procédé ci-dessus, sélection de 18 pics caractéristiques communs comme profil standard d'empreinte digitale.
